# EUROPÄISCHE PATENTSCHRIFT

(11) **EP 1 859 757 B1**
(45) Veröffentlichungstag und Bekanntmachung des Hinweises auf die Patenterteilung: **09.10.2013**
(21) Anmeldenummer: 07004776.6
(22) Anmeldetag: 08.03.2007
(51) Int. Cl.: A61C 13/00, A61C 13/09, A61C 5/10, C04B 33/14, A61K 6/00

(54) **Zusammensetzung und Verfahren zur Herstellung von gefärbten Rohlingen und dentalen Formteilen**
Composition and Method for manufacturing coloured blanks and dental moulded parts
Composition et Procédé destiné à la fabrication d'ébauches colorées et de pièces de formage dentaires

(30) Priorität: 23.05.2006 DE 102006024065; 13.09.2006 EP 06120608
(43) Veröffentlichungstag der Anmeldung: 28.11.2007
(60) Teilanmeldung: 11157242.6
(73) Patentinhaber: Ivoclar Vivadent AG, 9494 Schaan (LI)
(72) Erfinder: Ritzberger, Christian, 9472 Grabs (CH); Apel, Elke, Oberschan (CH); Höland, Wolfram, Prof. Dr., 9494 Schaan (LI); Rothbrust, Frank, 6820 Frastanz (AT); Kerschbaumer, Harald, 6833 Klaus (AT); Rheinberger, Volker M., Dr., 9490 Vaduz (LI)
(74) Vertreter: UEXKÜLL & STOLBERG

(56) Entgegenhaltungen:
- EP-A- 0 218 853
- WO-A-02/085242
- WO-A-2007/053084
- FR-A- 2 781 366
- JP-A- 8 033 650
- US-A- 5 011 403
- US-A- 5 263 858
- US-A- 5 656 564
- US-A1- 2003 125 189
- US-A1- 2004 152 034
- US-B1- 6 495 072

## Beschreibung

Die vorliegende Erfindung betrifft ein- oder mehrfarbige Formkörper, Rohlinge und dentale Formteile aus Oxidkeramiken, ein Verfahren zu deren Herstellung, deren Verwendung zur Herstellung von dentalen Restaurationsformteilen sowie eine Zusammensetzung, die zu deren Herstellung besonders geeignet ist.

Die Verwendung von Oxidkeramiken als Gerüstwerkstoff für dentale Restaurationen ist seit langem Stand der Technik. Dieses Material zeichnet sich durch eine ausgezeichnete Biokompatibilität und hervorragende mechanische Eigenschaften aus. Als Implantatwerkstoff und für Prothesen findet es seit vielen Jahren ebenfalls breite Anwendung. Dabei werden in den vergangenen Jahren insbesondere Keramiken auf der Basis von teilstabilisierten ZrO₂-Keramiken eingesetzt.

Die Verarbeitung dieser Keramiken in der Dentaltechnik erfolgt maschinell, wobei sich die Verarbeitung von nur teilgesinterten Keramiken mit einer Fräsbearbeitung mittels CAD/CAM-Bearbeitungsanlagen durchgesetzt hat. Dabei wird die Schwindung, die beim Dichtsintern von Formkörpern von einer Dichte von etwa 40 - 60 % auf eine Dichte von über 95 % auftritt, bei der maschinellen Bearbeitung bereits berücksichtigt. Dabei bezieht sich die Angabe der Dichte jeweils auf die theoretische Dichte.

Der Nachteil der ZrO₂-Keramiken besteht in der geringen Transluzenz und deren milchig-weißen Farbe. Damit erscheint eine nicht gefärbte und nicht beschichtete Restauration bzw. Restaurationsteil nur wie ein unnatürlicher Zahn. Somit sind Einfärbungen der ZrO₂-Keramik zur Anpassung an die Patientensituation für eine ästhetische Zahnrekonstruktion unabdingbar.

Ein besonders großer Nachteil von Sinterkeramiken nach dem Stand der Technik ist, dass sie keine Rohlinge für die CAD/CAM-Bearbeitung in offenporiger Form oder in dicht gesinterter Form ergeben, die eine Mehrfarbigkeit oder einen zonaren Aufbau unterschiedlicher, dem natürlichen Zahn entsprechenden Farben besitzen.

Aus dem Stand der Technik ist lediglich eine Reihe von technischen Lösungen für gefärbte, nicht mehrfarbige Rohlinge bekannt. Jedoch besitzen diese Lösungen den Nachteil, dass die natürliche Zahnfarbe, der Farbgradient, die Mehrfarbigkeit, die abgestufte Transluzenz und Farbhelligkeit nicht erreicht wurde. Diese bekannten Lösungen sind im Folgenden dargestellt:

Die Herstellung eines offenporigen gefärbten und weißen Y₂O₃-haltigen ZrO₂-Rohlings wird nach EP 1 210.054 aus Flüssigkeiten über die Kofällung aus Chloriden erzielt, die Zr-, Y-, Al-, Ga-, Ge, In, Fe-, Er- und Mn-Ionen erhalten. Mittels der Kofällung und anschließender Kalzinierung enthält das hergestellte Pulver die farbgebenden Ionen bereits vor der Formgebung. Als farbgebende Verbindungen werden Oxide aus der Gruppe Fe₂O₃, Er₂O₃ und MnO₂ ausgewählt. Der Nachteil dieser Erfindung besteht darin, dass ein sehr aufwendiges und umständliches Verfahren des Kofällungsprozesses mit anschließender Kalzinierung durchgeführt werden muss, um ein eingefärbtes Pulver zu erhalten. Das bedeutet, dass dies für jede einzelne Farbe vorgenommen werden muss.

In den folgenden Erfindungen werden monolithische Keramiken vorgestellt, die jeweils eine spezifische Farbe, also keine Mehrfarbigkeit erzielen lassen:

Die US 5,263,858 (Yoshida et al.) beschreibt die Herstellung von elfenbeinfarbenen Formkörpern für Dentalanwendungen (Brackets), indem bei der Herstellung der stabilisierten ZrO₂-Keramik vor dem Kalzinieren, färbende Verbindungen in Lösungen oder nach dem Kalzinieren pulverförmige Mischungen von färbenden Oxiden zugegeben werden. Um den gewünschten Elfenbein-Farbton zu erreichen, ist der Zusatz von Fe₂O₃, Pr₆O₁₁ und Er₂O₃ erforderlich. Dieser Prozess hat jedoch den Nachteil, dass er mehrstufig ist.

Des Weiteren ist aus dem Stand der Technik nach FR 2 781 366 bekannt, die färbenden Komponenten mit dem Ausgangspulver des ZrO₂ zu mischen; zu mahlen und gemeinsam zu sintern. Als färbende Oxide werden Fe₂O₃, CeO₂ und Bi₂O₃ genannt.

Die EP 0 955 267 erwähnt Gehalte von 5 - 49 Gew.-%" CeO₂, wodurch eine Farbgebung erzielt wird.

Zur Herstellung von vollständig kubisch stabilisiertem Zirkoniumdioxid im Lichtbogenofenprozess werden nach der EP 1 076 036 B1 einer ZrO₂-Quelle ein oder mehrere stabilisierende und färbende Oxide bzw. deren Vorläufer beigefügt. Die färbenden Oxide der Elemente Pr, Ce, Sm, Cd, Tb sind nach dem Sinterprozess in das Kristallgitter des ZrO₂ eingebaut.

Die US 5,656,564 betrifft die Herstellung von Zirkonoxid-Formkörpern, die Oxide der seltenen Erden, Boroxid, Aluminiumoxid und/ oder Siliciumoxid enthalten. Der Formkörper enthält das Zirkoniumdioxid als Mischphase von tetragonalem und monoklinem ZrO₂. Als Farboxide werden z.B. Oxide der Elemente Pr, Er und Yb in die Sinterkeramik eingebracht.

Weiterhin sind nach dem Stand der Technik technische Lösungen bekannt, die farbige Rohlinge durch Infiltration von Flüssigkeiten erzielen lassen. Diese technischen Lösungen besitzen jedoch den schwerwiegenden Nachteil, dass eine Farbgebung nach dem Vorsinterprozess erfolgt und damit in einen offenporigen Keramikkörper Flüssigkeiten eingebracht werden. Damit ist die Farbgebung nicht vollständig homogen und eine Mehrfarbigkeit ist ebenfalls nicht erzielbar.

Im Gegensatz zu Sinterkeramiken, wie ZrO₂ und Al₂O₃, ist in der Werkstoffgruppe der Glaskeramiken (DE 197 14 178 C2) ein Verfahren zur Herstellung von mehrfarbigen Glaskeramikrohlingen bekannt Die Herstellung von mehrfarbigen ZrO₂-Rohlingen wird in dieser Erfindung jedoch nicht erwähnt.

Ein Nachteil der bekannten Lösungen aus dem Stand der Technik ist, dass mehrfarbige Sinterkeramikrohlinge nicht hergestellt werden können. Außerdem sind die nach dem Stand der Technik genannten Lösungen sehr aufwendig und es treten Qualitätsprobleme auf. Letzteres trifft z.B. für die Infiltrationstechnik zu, bei der durch die nachträgliche Einfärbung eines teilgesinterten Rohlings oder eines geformten Dentalproduktes nur die Hohlräume (Poren) zwischen den teilweise zusammengesinterten Partikel des Ausgangspulvers durch die färbenden Ionen belegt werden können. Dadurch werden auch nur diskrete Bereiche der Oberfläche der Partikel mit einer Schicht der färbenden Oxide gefärbt, eine kontinuierliche Belegung der Oberfläche der Partikel des Ausgangspulvers ist nicht möglich. Ein weiterer großer Nachteil bei einer Infiltration besteht im Konzentrations-Gradienten der Einfärbung von außen nach innen. Wird ein poröser Körper in die Färbelösung eingelegt, so gibt die Ausgangslösung, beginnend von außen nach innen, einen Teil der gelösten färbenden Ionen ab und somit "verarmt "die Färbelösung an ihren färbenden Substanzen. Die Konsequenz daraus ist, dass außen eine höhere Konzentration der färbenden Ionen bzw. dann Oxide vorliegt als im Inneren des Formkörpers. Weiterhin kann mittels der Infiltrationstechnik nur eine bestimmte Eindringtiefe erreicht werden.

Die Aufgabe der Erfindung besteht nun darin, die Nachteile des oben beschriebenen Standes der Technik zu vermeiden und ein Oxid-Pulver, das die farbgebenden Verbindungen einheitlich verteilt enthält und für die Weiterverarbeitung zu einem dentalen Restaurationsteil aus diesem einheitlich gefärbten Oxid-Pulver geeignet ist, sowie einen Formkörper, einen offenporigen Rohling und ein dentales Formteil herzustellen, welche die farbgebenden Verbindungen einheitlich verteilt enthalten.

Weiterhin ist es Aufgabe der Erfindung ein- oder mehrfarbige Formteile, Rohlinge und dentale Formteile, die die farbgebenden Verbindungen in einem Gradienten oder zonaren Aufbau aufweisen, zur Verfügung zu stellen.

Gegenstand der Erfindung ist ein Verfahren gemäß Anspruch 25, bei welchen
a) ein Oxid-Pulver mit einer farbgebenden Substanz beschichtet,
b) das beschichtete Pulver gegebenenfalls klassiert und gegebenenfalls in eine Pressform gefüllt,
c) das gefärbte Pulver zu einem Formkörper gepresst und
d) der verpresste Formkörper zu einem Rohling gesintert wird, und
e) gegebenenfalls daraus das dentale Formteil geformt wird.

Die weitere Aufgabe der Erfindung wird durch ein- und mehrfarbige Formkörper, Rohlinge und dentale Formteile gemäß den Ansprüchen 19-24 gelöst.

Unter Schicht ist in diesem Sinne eine Einzelkomponente zu verstehen, die einen einfarbigen Formkörper, Rohling bzw. dentales Formteil ergibt. Der Begriff Schichten verdeutlicht den Farbverlauf als Farbgradient oder zonaren Aufbau eines mehrfarbigen Formkörpers, Rohlings bzw. mehrfarbigen dentalen Formteils.

Gegenstand der Erfindung ist ferner die Verwendung des ein-oder mehrfarbigen Formkörpers, Rohlings oder dentalen Formteils für die Herstellung von dentalen Restaurationsteilen gemäß Anspruch 44.

Gegenstand der Erfindung ist eine Zusammensetzung auf Basis von ZrO₂ gemäß Anspruch 1, die bereits mit sehr geringen Mengen an färbenden Substanzen zu sehr den natürlichen Zähnen ähnelnden dentalen Restauration verarbeitet werden können.

Bei den erfindungsgemäßen Verfahren erfolgt vor dem Verpressen der Rohlinge und damit vor dem Vorsintern bzw. Sintervorgang die Einfärbung der Oxidkeramik. Für die Herstellung von mehrfarbigen Formkörpern, Rohlingen bzw. dentalen Formteilen kann die Beschichtung mit weiteren farbgebenden Substanzen durchgeführt werden. Hierfür wird in einem ersten Schritt eine erste farbgebende Substanz auf ein ungefärbtes Oxidkeramik-Pulver aufgebracht. In einem zweiten Schritt wird sodann die nächste farbgebende Substanz auf weitere ungefärbte Oxidkeramik-Pulver aufgebracht. Je nach Zahl der gewünschten Farben kann in mehreren weiteren Schritten eine weitere Einfärbung, mit weiteren farbgebenden Substanzen auf weitere ungefärbte Oxidkeramik-Pulver, erfolgen. Im Ergebnis entstehen mithin unterschiedlich Farbverbindungen enthaltende Oxidpulverteilchen.

Die Umhüllung mit den farbgebenden Substanzen wird vorzugsweise in einem Wirbelschichtreaktor durchgeführt. Daneben sind erfindungsgemäß auch andere dem Fachmann bekannte Beschichtungsverfahren für Pulver einsetzbar.

Die geeigneten Wirbelschichtverfahren sind aus anderen Bereichen der Technik bekannt. Für die Einfärbung von Oxidkeramik-Pulvern, welche im Dentalbereich Verwendung finden, sind diese Verfahren noch nicht zum Einsatz gekommen.

In der Wirbelschicht (auch Fließbett genannt) wird eine durch ein Trägermedium (Gas oder Flüssigkeit) aufgelockerte Masse aus feinkörnigen Feststoffteilchen bewegt. Dazu wird eine ruhende Schüttung aus Oxidkeramik-Pulver mit einem durchschnittlichen Korndurchmesser der Granulate von 1 bis 100 µm, vorzugsweise von 30 bis 80 µm von einem Gas von unten nach oben durchströmt. Bei einer bestimmten Strömungsgeschwindigkeit (Lockerungspunkt, Wirbelpunkt) geht die Schüttung in die Wirbelschicht über. Pulver mit dem angegebenen mittleren Granulatdurchmesser sind daher bevorzugt. Bei der Wirbelbeschichtung kann sowohl das Bottom-Spray oder das Top-Spray-Verfahren verwendet werden.

Bei Ausbildung der Wirbelschicht wird die Gewichtskraft der Feststoffteilchen durch die entgegengesetzt gerichtete Strömungskraft des Trägermediums aufgehoben. Der Feststoff verhält sich dann flüssigkeitsähnlich, d.h., er kann während des Betriebes einfach zu- oder abgeführt werden. In der Wirbelschicht findet ein sehr intensiver Stoff- und Wärmeaustausch statt. Demgemäß kann erfindungsgemäß durch Einsatz eines Fluids, welches farbgebende Substanzen enthält, eine Einfärbung der Oxidkeramik-Pulver erreicht werden.

Als Oxidkeramik-Pulver werden oxidische Sinterkeramiken auf Basis von ZrO₂ eingesetzt.

Unter Sinterkeramiken werden Produkte verstanden, die aus kristallinen Rohstoffen in einem Wärmebehandlungsprozess durch Sintern erzeugt werden, wobei der Kristallanteil weitestgehend erhalten bleibt und nur ein geringer Anteil, meist deutlich weniger als 5 Vol.-% Glasphasenanteil zwischen den einzelnen Kristallen entsteht.

Erfindungsgemäß kann das ZrO₂ noch mit weiteren Metalloxiden dotiert sein. Beispielsweise ist eine Dotierung mit CeO₂, Y₂O₃, MgO oder CaO möglich. Weiterhin können HfO₂ und Al₂O₃ im ZrO₂-Pulver enthalten sein.

Als farbgebende Oxide werden Pr₂O₃, Fe₂O₃, Tb₂O₃ und Mn₂O₃ verwendet.

Als farbgebende Substanzen können vorzugsweise wässrige Lösungen verschiedener Salze zum Einsatz kommen. Bevorzugt sind wasserlösliche Salze von d- bzw. f-Elementen des Periodensystems. Besonders bevorzugt sind Nitrat- oder Chlorid-Hydrate. Beispiele für einsetzbare Salze sind Pr(NO₃) ₃·*5H₂O, Fe(NO₃)₃·*9H₂O oder Tb(NO₃)₃·*5H₂O.

Zusätzlich zu den farbgebenden Substanzen können geeignete wasserlösliche Bindemittel als Presshilfsmittel für das Pulver verwendet werden. Die Bindemittel werden vorzugsweise mit den genannten Salzen in Wasser gelöst und homogenisiert. Als Bindemittel ist Polyvinylalkohol besonders bevorzugt.

Weiter ist es bevorzugt Tensid zu dem Pulver zu zugeben, da dies überraschenderweise die Verpressbarkeit verbessert. Dies zeigt sich anhand einer höheren Pressdichte bei gleichem Druck. Besonders bevorzugt sind nicht-ionische oder amphotere Tenside, wie Polyglycolether oder Alkylsulfonate.

Die mit den farbgebenden Substanzen umhüllten Oxidkeramik-Pulver werden in einem zweiten Schritt vorzugsweise klassiert, z.B. gesiebt mit Sieben einer Maschenweite von < 90 µm. Dies wird insbesondere dann durchgeführt, wenn eine zu starke Agglomerierung des Pulvers vorliegt.

Das gegebenenfalls klassierte Pulver wird sodann üblicherweise in eine Pressform gegeben. Ist die Herstellung von mehrfarbigen Rohlingen vorgesehen, wird der vorstehend beschriebene erste Verfahrensschritt mit anderen färbenden Substanzen bzw. höheren Konzentrationen der färbenden Substanzen mehrfach ausgeführt, so dass unterschiedlich eingefärbte Pulver erzeugt werden. Diese unterschiedlich eingefärbten Pulver werden im zweiten Schritt nach dem Klassieren portionsweise in eine Pressform eingefüllt.

Das Pressen eines Formkörpers wird vorzugsweise kaltisostatisch bei Drücken von 50 bis 500 MPa, besonders bevorzugt bei 70 bis 300 MPa, ganz besonders bevorzugt bei 100 bis 200 MPa durchgeführt. Erfindungsgemäß ist auch ein uniaxiales Verpressen möglich.

In einem weiteren Verfahrensschritt wird der erhaltene Formkörper gesintert, insbesondere vorgesintert. Hierfür werden vorzugsweise Temperaturen von 800 bis 1300°C, besonders bevorzugt 1000°C bis 1200°C, ganz besonders bevorzugt von 1050°C bis 1150°C eingestellt. Durch den Vorsinterprozess wird vorzugsweise ein poröser Rohling mit einer Dichte von mindestens 30 %, vorzugsweise 40 bis 75 % der theoretischen Dichte der Oxid-Keramik erhalten. Die Dauer des Vorsinterns bei den angegebenen Temperaturen beträgt 1 bis 4 Stunden, vorzugsweise 1,5 bis 2,5 Stunden. Der gesamte Vorsinterschritt inklusive Aufheiz- und Abkühlvorgang dauert üblicherweise 38 bis 72 Stunden.

Während des Vorsinterprozesses erfolgt gleichzeitig das Entbindern des Formkörpers. Unter Entbindern wird das Ausbrennen der organischen Bestandteile, insbesondere der Bindemittel, verstanden. Diese Entbinderung kann jedoch auch in einem gesonderten Verfahrensschritt durchgeführt werden.

Der erhaltene ein- oder mehrfarbige Rohling wird beispielsweise im Dentallabor oder einer zahnärztlichen Klinik zu einem dentalen Formteil geformt. Dies kann vorzugsweise durch Fräsen oder Schleifen mittels einer CAD/CAM-Anlage erfolgen. Die -so erhaltene vergrößerte Vorform eines dentalen Formteil der Dentalrestauration wird anschließend bei einer Temperatur von vorzugsweise 1200°C bis 1600°C, besonders bevorzugt bei 1300°C bis 1550°C und ganz besonders bevorzugt bei 1400°C bis 1500°C dichtgesintert. Die Dauer des Dichtsinterns liegt vorzugsweise zwischen 5 Minuten und 2 Stunden. Ganz besonders bevorzugt sind 10 bis 30 Minuten. Der gesamte Dichtsinterprozess mit Aufheizen und Abkühlen dauert in der Regel etwa 8 Stunden. Der Fräsprozess wird derart durchgeführt, dass das dentale Formteil ein Übermaß aufweist, das die Schwindung des Formteils beim Dichtsintern berücksichtigt.

Das Dichtsintern kann auch mit Mikrowellenenergie herbeigeführt werden, was regelmäßig zur Verringerung der -Prozeßzeiten führt.

Das hergestellte dentale Formteil kann bereits die fertige dentale Restauration darstellen oder wird noch weiter verarbeitet, wie z.B. mit einer Verblendung versehen, um die fertige dentale Restauration zu ergeben.

Die in der beschriebenen Weise hergestellten Dental-Formteile zeichnen sich im Gegensatz zu den bisher aus dem Stand der Technik bekannten dentalen Formteilen durch eine hohe Farbhomogenität aus. Die farbgebenden Ionen liegen in wässriger Phase vor und werden insbesondere in der Wirbelschicht homogen auf die Oberfläche der Oxidkeramik-Pulver aufgetragen. Selbst bei sehr niedrigen Konzentrationen von ca. 0,001 Gew.-%, bezogen auf die Gesamtpulvermenge, wird eine homogene Beschichtung des Pulvers erreicht. Ebenso ist es erfindungsgemäß vorteilhaft, dass Mischfarben eingestellt werden und Rohlinge auf diese Art in mehrfarbiger Form hergestellt werden können.

Ein besonderer Schwerpunkt der Erfindung besteht in der Verwendung von mehrfarbigen Rohlingen in der Weise, dass nach dem Dichtsintern der ZrO₂-Keramik ein mehrfarbiges Produkt für die restaurative Zahnmedizin vorliegt. Diese Mehrfarbigkeit ist ganz besonders für mehrgliedrige Brücken äußerst vorteilhaft. Damit kann durch eine solche Dentalrestauration der natürliche Zahn in perfekter Weise wiedergegeben werden, denn dieser besteht nicht nur aus einer Farbe, sondern weist einen Farbgradienten und Transluzenzunterschiede sowie unterschiedliche Farbhelligkeiten innerhalb des Zahnes auf. Wird nun eine erfindungsgemäße mehrfarbige Brücke hergestellt, so können diese vorgenannten Anforderungen bzw. Eigenschaften eines natürlichen Zahnes durch einfache Weise sichergestellt werden. Diese Einfachheit der Herstellung von dentalen Formteilen, z.B. von komplizierten Dentalrestaurationen, wie mehrgliedrigen Brücken, zeigt sich erfindungsgemäß dadurch, dass der Zahntechniker auf die mehrfarbige Keramikbrücke nur wenige, oder sogar nur eine, Sinterkeramik- oder Sinterglaskeramikverblendungsschicht aufzubringen braucht, um beste Natürlichkeit zu erzielen. Auf ungefärbte Keramiken müssen mehrere Schichten als Verblendung und zur Erzielung spezieller Farbeffekte aufgebracht werden. Auch auf einfarbige Keramiken sind verschiedene Aufsinterungen erforderlich. Somit wird erfindungsgemäß effektiver als nach dem Stand der Technik gearbeitet.

Ein weiterer Vorteil besteht darin, dass eine gleichmäßige Verteilung von Poren nicht vorausgesetzt werden muss. Im Gegensatz dazu ist die Infiltrationstechnik abhängig von einer möglichst gleichmäßigen Verteilung der Poren, um wenigstens einigermaßen akzeptable Einfärbungen zu erreichen. Demgemäß werden nach dem Stand der Technik durch unterschiedliche Rauhigkeiten und nicht zugängliche Poren ganz verschiedene Einfärbequalitäten erreicht.

Vorteilhaft ist es erfindungsgemäß weiterhin, dass bei der erfindungsgemäßen Einfärbung der Oxidkeramik-Pulver durch die homogene Verteilung der farbgebenden Substanzen auf der Oberfläche der Pulver bzw. der Agglomerate aus den Primärpartikeln des Pulvers keine Anreicherung der farbgebenden Substanzen auftritt. Es wird somit zuverlässig eine Gefügestörung vermieden. Wird das vorstehend beschriebene Verfahren mit unterschiedlich gefärbten Oxid-Keramik-Pulvern durchgeführt, entsteht nach dem Pressen, Entbindern und Sintern ein mehrfarbiger Rohling, der einen Farbgradienten aufweist, der vorzugsweise dem eines natürlichen Zahns entspricht.

Weiterhin wird das Kornwachstum innerhalb der Keramik bei der Sinterung nicht negativ beeinflusst. Die Farbionenverteilung ist so günstig, dass weder mit dem menschlichen Auge noch mit einem Rasterelektronenmikroskop (REM) oder einem Transmissionenelektronenmikroskop (TEM) Farbgradienten oder auch Anreicherungen von Farbionen sichtbar oder analysierbar sind.

Erfindungsgemäß enthält der Rohling oder das dentale Formteil oder die daraus hergestellte dentale Restauration die nachstehend beschriebene erfindungsgemäße Zusammensetzung.

Die erfindungsgemäße Zusammensetzung ist auf Basis von ZrO₂, und enthält die folgenden Komponenten
Pr, berechnet als Pr₂O₃,
Fe, berechnet als Fe₂O₃,
Tb, berechnet als Tb₂O₃, und
Mn, berechnet als Mn₂O₃,
wobei diese in einer Gesamtmenge von 0,0001 bis 0,75 Gew.-% vorliegen.

Überraschenderweise hat es sich gezeigt, dass eine derartige Zusammensetzung trotz der überaus geringen Gesamtmenge von färbenden Komponenten, nämlich Pr, Fe, Tb und Mn, die Herstellung von Rohlingen, dentalen Formteilen und schließlich dentalen Restaurationen gestattet, die in der gewünschten Weise intensiv gefärbt sind, um das natürliche Zahnmaterial ausgezeichnet zu imitieren.

Die Komponenten Pr, Fe, Tb und Mn können in unterschiedlichen Oxidationsstufen in der Zusammensetzung vorhanden sein, wobei sie bevorzugt in einer von Null verschiedenen Oxidationsstufe vorhanden sind.

Es wird angenommen, dass bei der Herstellung der Zusammensetzung durch das oben angegebenen erfindungsgemäße Verfahren insbesondere nach durchgeführten Vorsinter- oder Dichtsinterschritten ein Einbau der Komponenten in Form von Ionen in das Kristallgitter des ZrO₂ erfolgt. Beispielsweise ist es vorstellbar, dass diese Ionen in Fehlstellen des ZrO₂-Gitters eingebaut werden.

Die Zusammensetzung liegt daher bevorzugt in gesinterter Form vor. Sie enthält darüber hinaus die angegebenen Komponenten bevorzugt in homogener Verteilung.

Es ist weiter bevorzugt, dass die Zusammensetzung die Komponenten in einer Gesamtmenge von 0,0001 bis 0,6 und insbesondere 0,0004 bis 0,37 Gew.-% enthält.

Weiter hat sich eine Zusammensetzung als besonders bevorzugt erwiesen, die die Komponenten in den folgenden Mengen enthält:

| Komponente | Mengen (Gew.-%) |
|---|---|
| Pr, berechnet als Pr₂O₃ | 0,0001 - 0,01 |
| Fe, berechnet als Fe₂O₃ | 0,005 - 0,5, |
| Tb, berechnet als Tb₂O₃ | 0,0001 - 0,1 |
| Mn, berechnet als Mn₂O₃ | 0,0001 - 0,1 |

Überdies existieren für die einzelnen Komponenten noch besonders bevorzugte Bereiche in der folgenden Weise:
Pr, berechnet als Pr₂O₃, in einer Menge von 0,0005 bis 0,01 Gew.-%
Fe, berechnet als Fe₂O₃, in einer Menge von 0,005 bis 0,4 Gew.-%
Tb, berechnet als Tb₂O₃, in einer Menge von 0,0001 bis 0,075 Gew.-%
Mn, berechnet als Mn₂O₃, in einer Menge von 0,0001 bis 0,075 Gew.-%.

Schließlich hat es sich überraschenderweise gezeigt, dass besondere Zusammensetzungen sehr vorteilhaft als Grundfarben bei der Herstellung von dentalen Restaurationen verwendet werden können. Es sind dies die im folgenden beschriebenen vier besonders bevorzugten Ausführungsformen der erfindungsgemäßen Zusammensetzung mit Angabe der enthaltenen Mengen der Komponenten:

| Komponente | Mengen (Gew.-%) |
|---|---|
| Pr, berechnet als Pr₂O₃ | 0,001 - 0,003 |
| Fe, berechnet als Fe₂O₃ | 0,005 - 0,04 |
| Tb, berechnet als Tb₂O₃ | 0,0005 - 0,004 |
| Mn, berechnet als Mn₂O₃ | 0,0001 - 0,0005 |
| | |

| Komponente | Mengen (Gew.-%) |
|---|---|
| Pr, berechnet als Pr₂O₃ | 0,002 - 0,004 |
| Fe, berechnet als Fe₂O₃ | 0,04 - 0,15 |
| Tb, berechnet als Tb₂O₃ | 0,0005 - 0,004 |
| Mn, berechnet als Mn₂O₃ | 0,0003 - 0,002 |
| | |

| Komponente | Mengen (Gew.-%) |
|---|---|
| Pr, berechnet als Pr₂O₃ | 0,003 - 0,006 |
| Fe, berechnet als Fe₂O₃ | 0,04 - 0,15 |
| Tb, berechnet als Tb₂O₃ | 0,0005 - 0,004 |
| Mn, berechnet als Mn₂O₃ | 0,0005 - 0,002 |
| | |

| Komponente | Mengen (Gew.-%) |
|---|---|
| Pr, berechnet als Pr₂O₃ | 0,003 - 0,006 |
| Fe, berechnet als Fe₂O₃ | 0,04 - 0,25 |
| Tb, berechnet als Tb₂O₃ | 0,0005 - 0,004 |
| Mn, berechnet als Mn₂O₃ | 0,001 -0,007 |

Die erfindungsgemäße Zusammensetzung kann darüber hinaus noch weitere Substanzen enthalten, wie insbesondere
Cr, berechnet als Cr₂O₃, in einer Menge von 0,0001 bis 0,1 Gew.-%.

Weiter ist es vorteilhaft, wenn die Zusammensetzung einen Stabilisator wie Y₂O₃ aufweist. Besonders bevorzugt sind daher Zusammensetzungen die 4 bis 8 Gew.-% Y₂O₃ enthalten.

Die Zusammensetzung enthält vorzugsweise auch bis zu 1 Gew.-% Al₂O₃. Dieses steigert die Stabilität der erfindungsgemäßen Zusammensetzung unter hydrothermalen Bedingungen, was insbesondere beim Einsatz der erfindungsgemäßen Zusammensetzung als Dentalmaterial von besonderer Bedeutung ist.

Die Zusammensetzung kann auch HfO₂ enthalten.

Besonders bevorzugt ist eine Zusammensetzung, die mehr als 95, insbesondere mehr als 98 Gew.-% ZrO₂ enthält. Bevorzugt sind auch Zusammensetzungen, bei denen ZrO₂ + HfO₂ + Y₂O₃ in mehr als 95 Gew.-% in der Zusammensetzung enthalten sind.

Die erfindungsgemäße Zusammensetzung wird insbesondere als Dentalmaterial verwendet. Die erfindungsgemäße Zusammensetzung kann insbesondere durch ein Verfahren hergestellt werden, bei dem ein Pulver auf ZrO₂-Basis mit einer Quelle der Komponenten, wie Verbindungen von Pr, Tb, Fe und Mn beschichtet wird, insbesondere mittels Wirbelbeschichtung.

Die Erfindung betrifft schließlich auch einen geformten Körper, der die erfindungsgemäße Zusammensetzung enthält und besonders solche, die eine homogene Verteilung von den Komponenten Pr, Fe, Tb und Mn innerhalb des gesamten Körpers und damit eine ausgezeichnete Farbhomogenität haben. Dieser geformte Körper liegt insbesondere als Rohling, dentales Formteil oder dentale Restauration vor, d.h. bevorzugt als gesinterter Formkörper. Der erfindungsgemäße geformte Körper kann insbesondere nach dem oben angegebenen erfindungsgemäßen Verfahren zur Herstellung von Rohlingen und dentalen Formteilen erzeugt werden.

Besonders bevorzugt sind erfindungsgemäße Rohlinge mit einer Dichte von 3,0 bis 3,5, insbesondere 3,1 bis 3,2 g/cm³.

Weiter ist ein geformter Körper bevorzugt, der mehrfarbig ist, wobei die Mehrfarbigkeit insbesondere durch mehrere unterschiedlich gefärbte Schichten hervorgerufen ist. Als besonders bevorzugt haben sich geformte Körper erwiesen, die mindestens zwei unterschiedlich gefärbte Schichten haben und vorzugsweise bis zu acht unterschiedlich gefärbte Schichten aufweisen.

Gerade mit einem geformter Körper, wie z.B. einem Rohling, mit wenigstens drei unterschiedlich gefärbten Schichten kann das natürliche Zahnmaterial sehr gut imitiert werden. Denn der natürliche Zahn kann grob in drei Bereiche, nämlich zervikal, zentral und inzisal mit jeweils unterschiedlichen Anforderungen an die optische Erscheinung eingeteilt werden. Somit wird die eine Schicht vorzugsweise dem zervikalen und die anderen beiden Schichten vorzugsweise dem zentralen und inzisalen Bereich des natürlichen Zahnes in ihrer optischen Erscheinung angepasst. Dabei hat es sich als besonders bevorzugt erwiesen, wenn mindestens eine Schicht des erfindungsgemäßen Körpers die erfindungsgemäße Zusammensetzung und insbesondere die Zusammensetzung der oben angegebenen vier besonderen Ausführungsformen der Erfindung enthält. Darüber hinaus ist es auch möglich, dass diese mindestens eine Schicht eine Mischung der oben angegebenen vier besonderen Ausführungsformen enthält und somit die durch diese Ausführungsformen repräsentierten Grundfarben abgewandelt werden.

Ein Vergleich mit herkömmlichen gefärbten Zusammensetzungen auf ZrO₂-Basis hat gezeigt, dass es die speziellen Komponenten der erfindungsgemäßen Zusammensetzungen selbst in sehr kleinen Mengen gestatten, eine angestrebte Farbintensität zu erreichen. Bei dem herkömmlichen System ist hierfür häufig eine deutlich höhere Menge an farbgebenden Komponenten erforderlich, was zu Störungen im Gefüge der daraus erzeugten geformten Körpern führt. Die demgegenüber bei den erfindungsgemäßen Zusammensetzungen eingesetzten geringen Mengen an farbgebenden Komponenten bewirken im wesentlichen keine Störung des Gefüges der daraus hergestellten geformten Körper, so dass diese und insbesondere die schließlich hergestellten Dentalrestaurationen ausgezeichnete physikalische Eigenschaften, wie Festigkeit und chemische Stabilität haben.

Die Erfindung betrifft daher schließlich auch die Verwendung der erfindungsgemäßen Zusammensetzung oder des erfindungsgemäßen Körpers zur Herstellung von dentalen Restaurationen. Die dentale Restauration ist dabei insbesondere eine Krone und ganz besonders bevorzugt eine Brücke. Gerade bei einer Brücke ist es von besonderer Bedeutung, wenn bei den zu ersetzenden natürlichen Zähnen vorliegende unterschiedliche Farben möglichst naturgetreu nachgeahmt werden können. Dies aber ist mit der erfindungsgemäßen Zusammensetzung und den daraus hergestellten geformten Körpern in ausgezeichneter Weise möglich, indem entsprechend unterschiedlich gefärbte Bereiche, wie unterschiedlich gefärbte Schichten vorgesehen werden.

Nachfolgend wird die Erfindung anhand von Beispielen näher erläutert.

### Beispiele

Alle im Rahmen der Beispiele angegebenen optischen Werte L, a und b sind nach den Normen DIN 5033 bzw. DIN 6174 bestimmt worden, indem ein Vergleich mit einer weißen Referenzprobe mit den Werten L*=93,11, a*=-0,64 und b*=4,22 durchgeführt wurde. Der Wert C stellt die Vektorsumme von a und b dar. Die Farbmessung erfolgte mit einem Spektrometer CM-3700d der Firma Konica-Minolta. Der CR-Wert wurde nach der Norm BS 5612 bestimmt und ist ein Maß für die Opazität.

Als Ausgangsmaterial für erfindungsgemäße Zusammensetzungen und daraus hergestellte Formkörper, Rohlinge und dentale Formteile wurden teilstabilisierte ZrO₂-Pulver der Fa. Tosoh, z.B. TZ-₃Y oder TZ-₃YSB-C, verwendet, die nachstehende Zusammensetzungen aufwiesen:

| | |
|---|---|
| ZrO₂ + HfO₂ + Y₂O₃ | > 99,0 Gew.-% |
| Y₂O₃ | 4,5 - 5,4 Gew. - % |
| HfO₂ | ≤ 5,0 Gew.-% |
| Al₂O₃ | 0 , 2 - 0,5 Gew.-% |
| andere Oxide | ≤ 0, 5 Gew.-% |
| Radioaktivität | < 200 Bq kg-¹ |
| Rohdichte | 3, 09 - 3,21 g cm⁻³ |

Als Färbelösung für die Wirbelschichtbeschichtung wurde eine wässrige Lösung verwendet, die sowohl die farbgebenden Ionen als auch einen wasserlöslichen Binder, wie Polyvinylalkohol, (z.B. Optapix PAF2 oder PAF35 der Firma Zschimmer & Schwarz) enthielt. Die Färbelösung kann ein oder mehrere farbgebende Ionen enthalten, um die gewünschte Färbung nach dem Dichtsintern zu bekommen. Die wässrige Färbelösung mit ca. 0.1 - 2 Gew.-% Binder (bezogen auf die Menge des zu beschichtenden Pulvers) enthielt die jeweiligen farbgebenden Ionen. Diese Lösung wurde etwa eine halbe Stunde homogenisiert (Magnetrührer oder ähnliches). Diese so hergestellte Färbelösung wurde anschließend mittels eines Wirbelschichtgranulierers komplett auf das zu beschichtende ZrO₂-Pulver aufgebracht. Dabei wurde das zu beschichtende Pulver mittels Druckluft (0.15 - 0.30 bar) in Schwebe (Wirbelbett) gehalten und gleichzeitig wurde die Färbelösung durch eine Düse, die oberhalb dieses Wirbelbettes angeordnet war, versprüht und auf das Pulver aufgetragen. Der Sprühdruck lag hierbei zwischen 2 bis 6 bar. Zusätzlich wurde die Druckluft, die zur Erhaltung des Wirbelbettes erforderlich war, auf etwa 30 bis 80 °C erhitzt, und sie konnte somit das Pulver während des Prozesses gleichzeitig trocknen.

### Herstellung eines einfarbigen ZrO₂-Rohlings:

ZrO₂-Pulver (TZ-3YSB-C) wurde mit einer wässrigen Färbelösung auf Basis von Fe(III)-, Pr- Cr- und Tb-Verbindungen beschichtet. Das so eingefärbte Pulver wurde in eine Pressform eingebracht und bei ca. 200 MPa kaltisostatisch verpresst. Dieser Formkörper wurde anschließend bei einer Temperatur von 1125°C über eine Zeit von ca. 120 min. zu einem Rohling vorgesintert und dann mittels CAD/CAM-Technologie bearbeitet. Abschließend wurde bei ca. 1500°C dichtgesintert. Das dentale Formteil zeigte nach dem Dichtsintern eine leicht gelbliche Färbung. Vorzugsweise wurden quaderförmige Rohlinge hergestellt, die bevorzugt folgende Abmessungen besaßen:

| | |
|---|---|
| Länge | ca. 15 bis ca. 60 mm |
| Breite | ca. 10 bis ca. 20 mm |
| Höhe | ca. 15 bis ca. 20 mm |

Die Herstellung von zylindrischen Rohlingen war aber ebenfalls möglich. Die mittels REM analysierte Kristallitgrösse des teilgesinterten Rohlings betrug ca. 200 bis 400 nm. Die Kristallitgrösse in der dicht gesinterten Restauration betrug ca. 400 bis 800 nm.

In der folgenden Tabelle ist die Zusammensetzung verschieden eingefärbter Rohlinge nach den Beispielen 1 bis 10 zusammen mit den optischen Werten nach dem Dichtsintern angegeben. Die Beispiele 1 bis 4 sind nicht erfindungsgemäß. Die Gehalte an den Komponenten sind dabei als Gehalte an dem jeweiligen dreiwertigen Oxid ausgedrückt. Dabei würden als Ausgangsmaterial ZrO₂-Pulver der Firma Tosoh, Japan, mit einem Gehalt an 4-8 Gew.-% Y₂O₃ eingesetzt. Die Pulver unterschieden sich in der Primärpartikelgröße, der spezifischen Oberfläche und dem Bindemittelanteil. Diese Pulver wurden wie oben beschreiben mit den färbenden Komponenten versehen.

| Beispiel | Zusammensetzung (TZ-3Y, TZ-3YSB oder TZ-3YSB-E) + folgende Komponenten | Optische Werte | | | | | |
|---|---|---|---|---|---|---|---|
| | | L* | a* | b* | C* | CR | Optisches Aussehen |
| 1 | 0,05 Gew.-% Pr₂O₃ | 84.02 | 2.37 | 25.74 | 25.85 | 94.33 | gelblich |
| | 0.0001 Gew.% Cr₂O₃ | | | | | | |
| | 0.001 Gew.-% Fe₂O₃ | | | | | | |
| | 0.0001 Gew.-% Tb₂O₃ | | | | | | |
| 2 | 0,1 Gew.-% Cr₂O₃ | 63.30 | 1.78 | 5.22 | 5.51 | 98.10 | dunkelgrau |
| | 0.0001 Gew.-% Tb₂O₃ | | | | | | |
| | 0.0001 Gew.-% Pr₂O₃ | | | | | | |
| | 0.001 Gew.-% Fe₂O₃ | | | | | | |
| 3 | 0,1 Gew.-% Fe₂O₃ | 81.39 | 1.80 | 17.23 | 17.33 | 97.97 | rötlich-braun |
| | 0.0001 Gew.-% Cr₂O₃ | | | | | | |
| | 0.0001 Gew.-% Pr₂O₃ | | | | | | |
| | 0.0001 Gew.-% Tb₂O₃ | | | | | | |
| 4 | 0.072 Gew.-% Fe₂O₃ | 79.19 | 1.87 | 18.64 | 18.74 | 99.47 | rötlich-braun |
| | 0.003 Gew.-% Cr₂O₃ | | | | | | (Zahnfarbe) |
| | 0.003 Gew.-% Pr₂O₃ | | | | | | |
| | 0.0001 Gew.-% Tb₂O₃ | | | | | | |
| 5 | 0,0001 Gew.-% Pr₂O₃ | 84.23 | 1.61 | 22.95 | 23.00 | 96.07 | gelblich |
| | 0.0001 Gew.-% Mn₂O₃ | | | | | | |
| | 0.0001 Gew.-% Fe₂O₃ | | | | | | |
| | 0.05 Gew.% Tb₂O₃ | | | | | | |
| 6 | 0,0001 Gew.-% Pr₂O₃ | 51.48 | 2.49 | -1.71 | 3.02 | 95.02 | anthrazit |
| | 0.1 Gew.-% Mn₂O₃ | | | | | | |
| | 0.0001 Gew.-% Fe₂O₃ | | | | | | |
| | 0.0001 Gew.-% Tb₂O₃ | | | | | | |
| 7 | 0,0040 Gew.-% Pr₂O₃ | 86.87 | -0.52 | 10.40 | 40.42 | 92.49 | leicht gelblich |
| | 0.0001 Gew.-% Mn₂O₃ | | | | | | |
| | 0.001 Gew.-% Fe₂O₃ | | | | | | |
| | 0.0001 Gew.-% Tb₂O₃ | | | | | | |
| 8 | 0,001 Gew.-% Pr₂O₃ | 72.24 | 6.10 | 22.10 | 22.92 | 98.59 | gelb-braun |
| | 0.0001 Gew.-% Mn₂O₃ | | | | | | |
| | 0.5 Gew.-% Fe₂O₃ | | | | | | |
| | 0.0001 Gew.-% Tb₂O₃ | | | | | | |
| 9 | 0,0006 Gew.-% Pr₂O₃ | 89.94 | -0.20 | 5.05 | 5.05 | 96.70 | creme-weiss |
| | 0.0001 Gew.-% Mn₂O₃ | | | | | | |
| | 0.0002 Gew.-% Fe₂O₃ | | | | | | |
| | 0.0001 Gew.-% Tb₂O₃ | | | | | | |
| 10 | 0,05 Gew.-% Pr₂O₃ | 53.09 | 1.54 | 4.35 | 4.62 | 99.49 | anthrazit |
| | 0.1 Gew.-% Mn₂O₃ | | | | | | |
| | 0.5 Gew.-% Fe₂O₃ | | | | | | |
| | 0.05 Gew.-% Tb₂O₃ | | | | | | |

### Beispiel 11

### Herstellung eines mehrfarbigen Rohlings auf ZrO₂-Basis:

Verschiedenfarbige Pulver (unterschiedliche Zahnfarben) wurden in eine Pressform je nach gewünschter Schichtdicke und Farbverlauf nacheinander homogen eingefüllt. Das Pulver wurde kaltisostatisch bei etwa 200 MPa verpresst und anschließend bei 1125°C für ca. 120 min. vorgesintert. Anschließend wurde aus dem Rohling eine Vorform eines dentalen Formteils herausgearbeitet und dichtgesintert. Das dentale Formteil wurde anschließend längs aufgeschnitten, und es zeigte sich ein Farbgradient, der so mittels Infiltrationstechnik nicht herstellbar ist. Dieser natürliche Farbgradient, also die Wiedergabe der optischen Eigenschaften des natürlichen Zahnes, wurde insbesondere bei einer 3-gliedrigen Seitenzahnbrücke deutlich. Unter Verwendung des erfindungsgemäßen Rohlings konnte ohne zusätzliche Beschichtungstechnik an dem dichtgesinterten dentalen Formteil der natürliche Farbverlauf rekonstruiert werden. Der Farbverlauf einer erfindungsgemäßen Seitenzahnbrücke ist dadurch gekennzeichnet, dass in der Fissur und am Zahnhals ein höheres. Chroma (eine intensivere Farbe) als im Gebiet der Zahnhöcker realisiert wird. Damit wurde eine wesentlich bessere Ästhetik gegenüber den Werkstoffen und Materialien des Standes der Technik erreicht. Weiterhin konnte der Zeitaufwand des Zahntechnikers für die Herstellung der komplett beschichteten Restauration reduziert werden.

Als Färbelösungen wurden wässrige Systeme mit löslichen ionogenen Verbindungen, bevorzugt Chloriden und Nitraten, verwendet.

Mit diesem Verfahren ließen sich Rohlinge mit einem Farbverlauf aus z.B. 2 bis zu 10 Farben in einer großen Homogenität als ein- und mehrfarbige Rohlinge herstellen.

### Beispiel 12

### Vergleichsbeispiel nach dem Stand der Technik:

Aus einem Brückenblock IPS e.max ZirCAD® wurde in einer CAD/CAM-Einheit (Sirona inLab®) ein 3-gliedriges Brückengerüst geschliffen. Der Schwindungsfaktor von etwa 20% je Raumachse wurde durch einen entsprechenden Vergrößerungsfaktor beim Schleifen berücksichtigt. Der Schleifprozess wurde nass durchgeführt, so dass vor dem Infiltrationsprozess das Gerüst getrocknet werden musste. Die Trocknung wurde über 2 Stunden bei ca. 80°C unter einer Infrarotlampe durchgeführt. Die nachbearbeiteten Gerüste (Abtrennen vom Handstück und Nachschleifen der Ränder) wurden mit nachstehenden Lösungen zur Einfärbung infiltriert (Angaben in Gew.-%), um Unterschiede in der Farbhomogenität zu ermitteln:

| Lösung | Fe(NO₃)₃*₉H₂O | H₂O | PEG 20000 | Ethanol |
|---|---|---|---|---|
| 1 | 4.2 | 76.6 | - | 19.2 |
| 2 | 4.8 | 54.4 | 27.2 | 13.6 |

| | | | | |
|---|---|---|---|---|
| PEG 20000: Polyethylenglycol (Fluka, Buchs, Schweiz) | | | | |

Ein Brückengerüst wurde für 2 min. in die Lösung 1 eingetaucht, die Infiltration erfolgte durch die Kapillarwirkung. Auf die Anwendung von Vakuum oder Überdruck wurde verzichtet. Nach der Infiltration wurde das Gerüst aus der Lösung entnommen und mittels Papiertuch trocken getupft, um die Überschüsse der Färbelösung von der Oberfläche zu entfernen. Anschließend erfolgte die Trocknung unter einer Infrarotlampe bei 80°C für etwa 2 Stunden. Bereits zu diesem Zeitpunkt zeigte sich eine Aufkonzentrierung der Fe-Ionen an exponierten Stellen des Gerüstes (z.B. Gerüsthöcker der Kaufläche, hohe Oberflächenkrümmung). Auch nach dem Dichtsintern bei 1500°C während 30 min. war die daraus resultierende Farbinhomogenität deutlich sichtbar. Eine gleichmäßige äußere Einfärbung war nicht realisierbar.

Ein weiteres Brückengerüst wurde zur Verbesserung der Farbhomogenität für 2 min. in die Färbelösung 2 getaucht. Die Nachbehandlung erfolgte wie vorstehend beschrieben. Es zeigte sich, dass der Nachteil der oberflächlichen Farbinhomogenität durch den Zusatz der organischen Komponente und damit eine Erhöhung der Viskosität der Färbelösung behoben werden konnte. Das Sintern erfolgte ebenfalls wie vorstehend beschrieben. Allerdings zeigte sich nach dem Zersägen des Brückengerüstes in Längsachse, dass die Einfärbung nur im äußeren Schichtbereich von ca. 0,6 bis 1,0 mm und damit keine homogene Einfärbung innerhalb des gesamten Gerüstes erfolgt war. Dies ist möglicherweise für eine Pfeilerkrone ausreichend, jedoch im Bereich der Verbinder und des Zwischengliedes der Brücke bestehen große Bedenken. Bei einem nachträglichen Beschleifen durch den Zahntechniker besteht die Gefahr, dass ungefärbte Bereiche teilweise freigelegt werden und der ursprünglich gewollte Effekt einer Einfärbung verloren geht.

Das Vergleichsbeispiel zeigt, dass bei der Anwendung der Infiltration als Einfärbeverfahren nur eine oberflächliche Einfärbung erreichbar ist oder eine vollständige Durchfärbung mit nur inhomogener, lokal unterschiedlich starker Farbgebung erzielt wird (Aufkonzentrierung der Farbionen). Erfindungsgemäß werden diese Nachteile vermieden. Es ist stets eine homogene Einfärbung des gesamten Rohlings feststellbar.

Weiterhin hat es sich als vorteilhaft herausgestellt, wenn nanoskaliges ZrO₂-Pulver als Ausgangsmaterial (Primärpartikelgröße zwischen 5 und 50 nm und eine spezifische Oberfläche von > 100 m^{2*}g⁻¹) für die Herstellung der Blöcke verwendet wird. Dieses zeigt nach der Bearbeitung zu einem Brückengerüst eine deutlich reduzierte Temperatur beim Dichtsintern. So wurden, in Abhängigkeit der verwendeten Materialien, Temperaturen von unter 1250°C erreicht, was ein Dichtsintern in üblichen Dentalbrennöfen erlaubt. Zur Herstellung von eingefärbten Blöcken (ein- und mehrfarbig) wurden die vorstehend verwendeten gefärbten Pulver als so genanntes Farbkonzentrat dem nanoskaligen ZrO₂-Pulver in einem Anteil von 0,0001 bis 2,0 Gew.-% zugesetzt. Eine Temperaturänderung beim Dichtsintern ist nicht erforderlich.

### Beispiele 13 bis 17

Es wurden in der oben für die Beispiele 1 bis 10 angegebenen Weise weitere besonders bevorzugte erfindungsgemäße Zusammensetzungen hergestellt und zu Rohlingen, dentalen Formteilen und Dentalrestaurationen weiterverarbeitet. Die Anteile an den Komponenten, berechnet als jeweiliges dreiwertiges Oxid, sowie der Gesamtanteil dieser Farbkomponenten und das Vorliegen eines bei der Verpressung zu geformten Körpern eventuell vorhandenen Additivs sind in der unten angeführten Tabelle 1 aufgeführt. Die Anteile sind dabei als mg Komponenten zu kg gesamte Zusammensetzung angegeben.

Bei dem Tensid handelte es sich um Polyglycolether oder Alkylsulfonate.

Bei diesen Zusammensetzungen und insbesondere denen gemäß Beispiel 13 und 17 handelt es sich um solche, die als typische Dentalfarben eingesetzt werden können. Überraschenderweise können diese Zusammensetzungen trotz der sehr geringen Mengen an farbgebenden Komponenten zu intensiv gefärbten Dentalrestaurationen, wie insbesondere Kronen und Brücken, verarbeitet werden.

Die optische Eigenschaften von daraus hergestellten gefärbten dichtgesinterten Rohlingen sind in der nachstehenden Tabelle 2 aufgeführt.

**Tabelle 1**

| Beispiel | Fe₂O₃ mg/kg | Pr₂O₃ mg/kg | Tb₂O₃ mg/kg | Mn₂O₃ mg/kg | Gesamtant eil FArbkomp. mg/kg | Additive |
|---|---|---|---|---|---|---|
| 13 | 329 | 15 | 7 | 1 | 352 | Optapix PAF35 |
| 14 | 1 | 1 | 1 | 1000 | 1003 | Tensid |
| 15 | 5000 | 10 | 1 | 1 | 5012 | Tensid |
| 16 | 1 | 1 | 500 | 1 | 503 | Tensid |
| 17 | 1000 | 25 | 6 | 10 | 1041 | Tensid |

**Tabelle 2**

| Beispiel | L | a | b | C | CR |
|---|---|---|---|---|---|
| 13 | 87.76 | -1.02 | 10.22 | - | 94.62 |
| 14 | 51.48 | 2.49 | -1.71 | 3.02 | 95.02 |
| 15 | 72.24 | 6.10 | 22.10 | 22.92 | 98.59 |
| 16 | 84.23 | 1.61 | 22.95 | 23.00 | 96.07 |
| 17 | 82.15 | 0.48 | 13.60 | - | 99.72 |

## Patentansprüche

1. Zusammensetzung auf Basis von ZrO₂, die die folgenden Komponenten
Pr, berechnet als Pr₂O₃,
Fe, berechnet als Fe₂O₃,
Tb, berechnet als Tb₂O₃, und
Mn, berechnet als Mn₂O₃,
enthält, wobei diese in einer Gesamtmenge von 0,0001 bis 0,75 Gew.-% vorliegen.

2. Zusammensetzung nach Anspruch 1, die die Komponenten in einer Gesamtmenge von 0,0001 bis 0,6 Gew.-% enthält.

3. Zusammensetzung nach einem der Ansprüche 1 oder 2, die die Komponenten in einer Gesamtmenge von 0,0004 bis 0,37 Gew.-% enthält.

4. Zusammensetzung nach einem der Ansprüche 1 bis 3, die die Komponenten in den folgenden Mengen enthält:
| Komponente | Mengen (Gew.-%) |
|---|---|
| Pr, berechnet als Pr₂O₃ | 0,0001 - 0,01 |
| Fe, berechnet als Fe₂O₃ | 0,005 - 0,5 |
| Tb, berechnet als Tb₂O₃ | 0,0001 - 0,1 |
| Mn, berechnet als Mn₂O₃ | 0,0001 - 0,1 |

5. Zusammensetzung nach einem der Ansprüche 1 bis 4, die Pr, berechnet als Pr₂O₃, in einer Menge von 0,0005 bis 0,01 Gew.-% enthält.

6. Zusammensetzung nach einem der Ansprüche 1 bis 5, die Fe, berechnet als Fe₂O₃, in einer Menge von 0,005 bis 0,4 Gew.-% enthält.

7. Zusammensetzung nach einem der Ansprüche 1 bis 6, die Tb, berechnet als Tb₂O₃, in einer Menge von 0,0001 bis 0,075 Gew.-% enthält.

8. Zusammensetzung nach einem der Ansprüche 1 bis 7, die Mn, berechnet als Mn₂O₃, in einer Menge von 0,0001 bis 0,075 Gew.-% enthält.

9. Zusammensetzung nach einem der Ansprüche 1 bis 8, die die Komponenten in den folgenden Mengen enthält:
| Komponente | Mengen (Gew.-%) |
|---|---|
| Pr, berechnet als Pr₂O₃ | 0,001 - 0,003 |
| Fe, berechnet als Fe₂O₃ | 0,005 - 0,04 |
| Tb, berechnet als Tb₂O₃ | 0,0005 - 0,004 |
| Mn, berechnet als Mn₂O₃ | 0,0001 - 0,0005 |

10. Zusammensetzung nach einem der Ansprüche 1 bis 8, die die Komponenten in den folgenden Mengen enthält:
| Komponente | Mengen (Gew.-%) |
|---|---|
| Pr, berechnet als Pr₂O₃ | 0,002 - 0,004 |
| Fe, berechnet als Fe₂O₃ | 0,04 - 0,15 |
| Tb, berechnet als Tb₂O₃ | 0,0005 - 0,004 |
| Mn, berechnet als Mn₂O₃ | 0,0003 - 0,002 |

11. Zusammensetzung nach einem der Ansprüche 1 bis 8, die die Komponenten in den folgenden Mengen enthält:
| Komponente | Mengen (Gew.-%) |
|---|---|
| Pr, berechnet als Pr₂O₃ | 0,003 - 0,006 |
| Fe, berechnet als Fe₂O₃ | 0,04 - 0,15 |
| Tb, berechnet als Tb₂O₃ | 0,0005 - 0,004 |
| Mn, berechnet als Mn₂O₃ | 0,0005 - 0,002 |

12. Zusammensetzung nach einem der Ansprüche 1 bis 8, die die Komponenten in den folgenden Mengen enthält:
| Komponente | Mengen (Gew.-%) |
|---|---|
| Pr, berechnet als Pr₂O₃ | 0,003 - 0,006 |
| Fe, berechnet als Fe₂O₃ | 0,04 - 0,25 |
| Tb, berechnet als Tb₂O₃ | 0,0005 - 0,004 |
| Mn, berechnet als Mn₂O₃ | 0,001 - 0,007 |

13. Zusammensetzung nach einem der Ansprüche 1 bis 12, die 4 bis 8 Gew.-% Y₂O₃ enthält.

14. Zusammensetzung nach einem der Ansprüche 1 bis 13, die bis zu 1 Gew.-% Al₂O₃ enthält.

15. Zusammensetzung nach einem der Ansprüche 1 bis 14, die zu 98,0 bis 99,9999 Gew.-% aus nanoskaligem ZrO₂-Pulver besteht.

16. Zusammensetzung nach Anspruch 15, bei der das nanoskalige ZrO₂-Pulver eine Primärpartikelgröße zwischen 5 und 50 nm und eine spezifische Oberfläche von ≥ 100 m²*g⁻¹ aufweist.

17. Zusammensetzung nach einem der Ansprüche 1 bis 16, die durch ein Verfahren herstellbar ist, bei dem ein Pulver auf ZrO₂-Basis mit einer Quelle der Komponenten Pr, Fe, Tb und Mn beschichtet wird.

18. Zusammensetzung nach Anspruch 17, bei der die Beschichtung mittels Wirbelbeschichtung erfolgt.

19. Geformter Körper, der die Zusammensetzung nach einem der Ansprüche 1 bis 18 enthält.

20. Geformter Körper nach Anspruch 19, der als Rohling, dentales Formteil oder dentale Restauration vorliegt.

21. Geformter Körper nach Anspruch 19 oder 20, der mehrfarbig ist.

22. Geformter Körper nach Anspruch 21, bei dem die Mehrfarbigkeit durch unterschiedlich gefärbte Schichten hervorgerufen ist.

23. Geformter Körper nach Anspruch 22, der mindestens 2 und vorzugsweise bis zu 8 unterschiedlich gefärbte Schichten hat.

24. Geformter Körper nach Anspruch 22 oder 23, bei dem mindestens eine Schicht die Zusammensetzung nach einem der Ansprüche 1-18, und insbesondere die Zusammensetzung nach einem der Ansprüche 9 bis 12, enthält.

25. Verfahren zur Herstellung von Rohlingen und dentalen Formteilen, die farbgebende Verbindungen enthalten und wie in Ansprüchen 20 bis 24 definiert sind, bei dem
a) ein ZrO₂ enthaltendes Oxid-Pulver mit einer farbgebenden Substanz beschichtet wird, wobei als farbgebende Substanz eine Quelle der Komponenten Pr, Fe, Tb und Mn eingesetzt wird,
b) das beschichtete Pulver gegebenenfalls klassiert und gegebenenfalls in eine Pressform gefüllt wird,
c) das gefärbte Pulver zu einem Formkörper gepresst wird, und
d) der verpresste Formkörper zu einem Rohling gesintert wird und
e) gegebenenfalls daraus das dentale Formteil geformt wird.

26. Verfahren nach Anspruch 25, **dadurch gekennzeichnet, dass** nach Schritt a) weitere ungefärbte Oxid-Pulver mit wenigstens einer weiteren farbgebenden Substanz beschichtet und nacheinander in eine Pressform gefüllt werden.

27. Verfahren nach Anspruch 25 oder 26, **dadurch gekennzeichnet, dass** als farbgebende Substanzen wässrige Lösungen von farbgebenden Metallsalzen von d- bzw. f-Elementen des Periodensystems eingesetzt werden.

28. Verfahren nach einem der Ansprüche 25 bis 27, **dadurch gekennzeichnet, dass** als farbgebende Substanzen Nitrat-Hydrate oder Chlorid-Hydrate eingesetzt werden.

29. Verfahren nach einem der Ansprüche 25 bis 28, **dadurch gekennzeichnet, dass** als farbgebende Substanzen Pr(NO₃)₃*5H₂O, Fe(NO₃)₃*9H₂O, Tb(NO₃)₃*5H₂O oder CrCl₃*6H₂O eingesetzt werden.

30. Verfahren nach einem der Ansprüche 25 bis 29, **dadurch gekennzeichnet, dass** die farbgebenden Substanzen mit einem wasserlöslichen Bindemittel in Wasser gelöst und homogenisiert werden.

31. Verfahren nach einem der Ansprüche 25 bis 30, **dadurch gekennzeichnet, dass** die Beschichtung der Oxid-Pulver in einem Wirbelschichtreaktor erfolgt.

32. Verfahren nach einem der Ansprüche 25 bis 31, **dadurch gekennzeichnet, dass** die Oxid-Pulver mit stabilisierenden Oxiden des Ce, Y, Al, Ca und/oder Mg dotiert sind.

33. Verfahren nach einem der Ansprüche 25 bis 32, **dadurch gekennzeichnet, dass** das Pressen zu Formkörpern bei Drücken von 50 bis 500 MPa erfolgt.

34. Verfahren nach einem der Ansprüche 25 bis 33, **dadurch gekennzeichnet, dass** der Formkörper zunächst vorgesintert wird.

35. Verfahren nach einem der Ansprüche 25 bis 34, **dadurch gekennzeichnet, dass** der Formkörper zunächst bei einer Temperatur von 800 bis 1300°C zu einem Rohling vorgesintert wird.

36. Verfahren nach einem der Ansprüche 34 oder 35, **dadurch gekennzeichnet, dass** die Dauer des Vorsinterprozesses 1 bis 4 Stunden beträgt.

37. Verfahren nach einem der Ansprüche 34 bis 36, **dadurch gekennzeichnet, dass** das dentale Formteil mittels materialabtragendem Verfahren aus dem vorgesinterten Rohling hergestellt und bei einer Temperatur von 1200 bis 1600°C dichtgesintert wird.

38. Verfahren nach einem der Ansprüche 25 bis 37, **dadurch gekennzeichnet, dass** die Dauer des Dichtsinterns 5 Minuten bis 2 Stunden beträgt.

39. Verfahren nach einem der Ansprüche 25 bis 38, **dadurch gekennzeichnet, dass** als Oxidkeramik zur Herstellung der Formkörper und der Rohlinge ein Pulver eingesetzt wird, das zu 98,0 bis 99,9999 Gew.-% aus nanoskaligem ZrO₂-Pulver besteht.

40. Verfahren nach Anspruch 39, **dadurch gekennzeichnet, dass** das nanoskalige ZrO₂-Pulver eine spezifische Oberfläche von ≥ 100 m²g⁻¹ hat und 0,0001 bis 2 Gew.-% einer anderen oxidischen Komponente enthält.

41. Verfahren nach Anspruch 40, **dadurch gekennzeichnet, dass** als weitere oxidische Komponente eine ZrO₂-Verbindung mit einer spezifischen Oberfläche < 100 m²g⁻¹, eine gefärbte ZrO₂-Verbindung oder ein farbgebendes Oxid oder eine Mischung aus farbgebenden Oxiden eingesetzt werden.

42. Verfahren nach einem der Ansprüche 39 bis 41, **dadurch gekennzeichnet, dass** das dentale Formteil mittels materialabtragendem Verfahren aus dem vorgesinterten Rohling hergestellt und bei einer Temperatur von 1000 bis 1250°C dichtgesintert wird.

43. Verwendung der Zusammensetzung nach einem der Ansprüche 1 bis 18 als Dentalmaterial.

44. Verwendung der Zusammensetzung nach einem der Ansprüche 1 bis 18 oder des geformten Körpers nach einem der Ansprüche 19 bis 24 zur Herstellung von dentalen Restaurationen.

45. Verwendung nach Anspruch 44, wobei die dentale Restauration eine Krone und insbesondere eine Brücke ist.

## Claims

1. Composition on the basis of ZrO₂ which contains the following components
Pr, calculated as Pr₂O₃,
Fe, calculated as Fe₂O₃,
Tb, calculated as Tb₂O₃, and
Mn, calculated as Mn₂O₃,
wherein these are present in a total quantity of 0.0001 to 0.75 wt.-%.

2. Composition according to claim 1 which contains the components in a total quantity of 0.0001 to 0.6 wt.-%.

3. Composition according to any one of claims 1 or 2 which contains the components in a total quantity of 0.0004 to 0.37 wt.-%.

4. Composition according to any one of claims 1 to 3 which contains the components in the following quantities:
| Component | Quantities (wt.-%) |
|---|---|
| Pr, calculated as Pr₂O₃ | 0.0001 - 0.01 |
| Fe, calculated as Fe₂O₃ | 0.005 - 0.5 |
| Tb, calculated as Tb₂O₃ | 0.0001 - 0.1 |
| Mn, calculated as Mn₂O₃ | 0.0001 - 0.1 |

5. Composition according to any one of claims 1 to 4 which contains Pr, calculated as Pr₂O₃, in a quantity of 0.0005 to 0.01 wt.-%.

6. Composition according to any one of claims 1 to 5 which contains Fe, calculated as Fe₂O₃, in a quantity of 0.005 to 0.4 wt.-%.

7. Composition according to any one of claims 1 to 6 which contains Tb, calculated as Tb₂O₃, in a quantity of 0.0001 to 0.075 wt.-%.

8. Composition according to any one of claims 1 to 7 which contains Mn, calculated as Mn₂O₃, in a quantity of 0.0001 to 0.075 wt.-%.

9. Composition according to any one of claims 1 to 8 which contains the components in the following quantities:
| Component | Quantities (wt.-%) |
|---|---|
| Pr, calculated as Pr₂O₃ | 0.001 - 0.003 |
| Fe, calculated as Fe₂O₃ | 0.005 - 0.04 |
| Tb, calculated as Tb₂O₃ | 0.0005 - 0.004 |
| Mn, calculated as Mn₂O₃ | 0.0001 - 0.0005 |

10. Composition according to any one of claims 1 to 8 which contains the components in the following quantities:
| Component | Quantities (wt.-%) |
|---|---|
| Pr, calculated as Pr₂O₃ | 0.002 - 0,004 |
| Fe, calculated as Fe₂O₃ | 0.04 - 0.15 |
| Tb, calculated as Tb₂O₃ | 0.0005 - 0.004 |
| Mn, calculated as Mn₂O₃ | 0.0003 - 0.002 |

11. Composition according to any one of claims 1 to 8 which contains the components in the following quantities:
| Component | Quantities (wt.-%) |
|---|---|
| Pr, calculated as Pr₂O₃ | 0.003 - 0.006 |
| Fe, calculated as Fe₂O₃ | 0.04 - 0.15 |
| Tb, calculated as Tb₂O₃ | 0.0005 - 0.004 |
| Mn, calculated as Mn₂O₃ | 0.0005 - 0.002 |

12. Composition according to any one of claims 1 to 8 which contains the components in the following quantities:
| Component | Quantities (wt.-%) |
|---|---|
| Pr, calculated as Pr₂O₃ | 0.003 - 0.006 |
| Fe, calculated as Fe₂O₃ | 0.04 - 0.25 |
| Tb, calculated as Tb₂O₃ | 0.0005 - 0.004 |
| Mn, calculated as Mn₂O₃ | 0.001 - 0.007 |

13. Composition according to any one of claims 1 to 12 which contains 4 to 8 wt.-% Y₂O₃.

14. Composition according to any one of claims 1 to 13 which contains up to 1 wt.-% Al₂O₃.

15. Composition according to any one of claims 1 to 14 which consists to 98.0 to 99.9999 wt.-% of nanoscale ZrO₂ powder.

16. Composition according to claim 15, wherein the nanoscale ZrO₂ powder has a primary particle size between 5 and 50 nm and a specific surface area of ≥ 100 m²g⁻¹.

17. Composition according to any one of claims 1 to 16 which is preparable by a process wherein a powder based on ZrO₂ is coated with a source of the components Pr, Fe, Tb and Mn.

18. Composition according to claim 17, wherein the coating is effected by fluidized-bed coating.

19. Shaped body which comprises the composition according to any one of claims 1 to 18.

20. Shaped body according to claim 19 which is present as a blank, dental shaped part or dental restoration.

21. Shaped body according to claim 19 or 20 which is multi-coloured.

22. Shaped body according to claim 21 in which the multi-coloration is brought about by differently coloured layers.

23. Shaped body according to claim 22 which has at least 2 and preferably up to 8 differently coloured layers.

24. Shaped body according to claim 22 or 23 in which at least one layer contains the composition according to any one of claims 1 to 18, and in particular the composition according to any one of claims 9 to 12.

25. Process for the preparation of blanks and dental shaped parts which contain colouring compounds and are as defined in claims 20 to 24, in which
a) a ZrO₂-containing oxide powder is coated with a colouring substance, wherein a source of the components Pr, Fe, Tb and Mn is used as coloring substance,
b) the coated powder is optionally graded and optionally filled into a compression mould,
c) the coloured powder is compressed to give a shaped body, and
d) the compressed shaped body is sintered to produce a blank and
e) optionally the dental shaped part is formed therefrom.

26. Process according to claim 25, wherein after step a) further uncoloured oxide powders are coated with at least one further colouring substance and filled successively into a compression mould.

27. Process according to claim 25 or 26, wherein aqueous solutions of colouring metal salts of d- or f-elements of the periodic table are used as colouring substances.

28. Process according to any one of claims 25 to 27, wherein nitrate hydrates or chloride hydrates are used as colouring substances.

29. Process according to any one of claims 25 to 28, wherein Pr(NO₃)₃ *5H₂O, Fe(NO₃)₃ *9H₂O, Tb(NO₃)₃ *5H₂O or CrCl₃*6H₂O are used as colouring substances.

30. Process according to any one of claims 25 to 29, wherein the colouring substances are dissolved in water with a water-soluble binder and homogenized.

31. Process according to any one of claims 25 to 30, wherein the coating of the oxide powders takes place in a fluidized-bed reactor.

32. Process according to any one of claims 25 to 31, wherein the oxide powders are doped with stabilizing oxides of Ce, Y, Al, Ca and/or Mg.

33. Process according to any one of claims 25 to 32, wherein the compression into shaped bodies takes place at pressures of 50 to 500 MPa.

34. Process according to any one of claims 25 to 33, wherein the shaped body is pre-sintered first.

35. Process according to any one of claims 25 to 34, wherein the shaped body is pre-sintered first at a temperature of 800 to 1300°C to produce a blank.

36. Process according to any one of claims 34 or 35, wherein the duration of the pre-sintering process is 1 to 4 hours.

37. Process according to any one of claims 34 to 36, wherein the dental shaped part is prepared by means of a material-removing process from the pre-sintered blank and is dense-sintered at a temperature of 1200 to 1600°C.

38. Process according to any one of claims 25 to 37, wherein the duration of the dense sintering is 5 minutes to 2 hours.

39. Process according to any one of claims 25 to 38, wherein there is used as oxide ceramic for the preparation of the shaped bodies and the blanks a powder which consists to 98.0 to 99.9999 wt.-% of nanoscale ZrO₂ powder.

40. Process according to claim 39, wherein the nanoscale ZrO₂ powder has a specific surface area of ≥ 100 m²g⁻¹ and contains 0.0001 to 2 wt.-% of another oxidic component.

41. Process according to claim 40, wherein there is used as further oxidic component a ZrO₂ compound with a specific surface area < 100 m²g⁻¹, a coloured ZrO₂ compound or a colouring oxide or a mixture of colouring oxides.

42. Process according to any one of claims 39 to 41, wherein the dental shaped part is prepared by means of a material-removing process from the pre-sintered blank and is dense-sintered at a temperature of 1000 to 1250°C.

43. Use of the composition according to any one of claims 1 to 18 as dental material.

44. Use of the composition according to any one of claims 1 to 18 or of the shaped body according to any one of claims 19 to 24 for the preparation of dental restorations.

45. Use according to claim 44, wherein the dental restoration is a crown and in particular a bridge.

## Revendications

1. Composition à base de ZrO₂, qui contient les composants suivants :
Pr, calculé comme Pr₂O₃,
Fe, calculé comme Fe₂O₃,
Tb, calculé comme Tb₂O₃, et
Mn, calculé comme Mn₂O₃,
ces composants étant présents en une quantité totale allant de 0,0001 à 0,75 % en poids.

2. Composition selon la revendication 1, qui contient les composants en une quantité totale allant de 0,0001 à 0,6 % en poids.

3. Composition selon l'une quelconque des revendications 1 ou 2, qui contient les composants en une quantité totale allant de 0,0004 à 0,37 % en poids.

4. Composition selon l'une quelconque des revendications 1 à 3, qui contient les composants en les quantités totales suivantes :
| Composants | Quantités (% en poids) |
|---|---|
| Pr, calculé comme Pr₂O₃ | 0,0001 - 0,01 |
| Fe, calculé comme Fe₂O₃ | 0,005 - 0,5 |
| Tb, calculé comme Tb₂O₃ | 0,0001 - 0,1 |
| Mn, calculé comme Mn₂O₃ | 0,0001 - 0,1 |

5. Composition selon l'une quelconque des revendications 1 à 4, qui contient Pr, calculé comme Pr₂O₃, en une quantité allant de 0,0005 à 0,01 % en poids.

6. Composition selon l'une quelconque des revendications 1 à 5, qui contient Fe, calculé comme Fe₂O₃, en une quantité allant de 0,005 à 0,4 % en poids.

7. Composition selon l'une quelconque des revendications 1 à 6, qui contient Tb, calculé comme Tb₂O₃, en une quantité allant de 0,0001 à 0,075 % en poids.

8. Composition selon l'une quelconque des revendications 1 à 7, qui contient Mn, calculé comme Mn₂O₃, en une quantité allant de 0,0001 à 0,075 % en poids.

9. Composition selon l'une quelconque des revendications 1 à 8, qui contient les composants en les quantités suivantes :
| Composants | Quantités (% en poids) |
|---|---|
| Pr, calculé comme Pr₂O₃ | 0,001 - 0,003 |
| Fe, calculé comme Fe₂O₃ | 0,005 - 0,04 |
| Tb, calculé comme Tb₂O₃ | 0,0005 - 0,004 |
| Mn, calculé comme Mn₂O₃ | 0,0001 - 0,0005 |

10. Composition selon l'une quelconque des revendications 1 à 8, qui contient les composants en les quantités suivantes :
| Composants | Quantités (% en poids) |
|---|---|
| Pr, calculé comme Pr₂O₃ | 0,002 - 0,004 |
| Fe, calculé comme Fe₂O₃ | 0,04 - 0,15 |
| Tb, calculé comme Tb₂O₃ | 0,0005 - 0,004 |
| Mn, calculé comme Mn₂O₃ | 0,0003 - 0,002 |

11. Composition selon l'une quelconque des revendications 1 à 8, qui contient les composants en les quantités suivantes :
| Composants | Quantités (% en poids) |
|---|---|
| Pr, calculé comme Pr₂O₃ | 0,003 - 0,006 |
| Fe, calculé comme Fe₂O₃ | 0,04 - 0,15 |
| Tb, calculé comme Tb₂O₃ | 0,0005 - 0,004 |
| Mn, calculé comme Mn₂O₃ | 0,0005 - 0,002 |

12. Composition selon l'une quelconque des revendications 1 à 8, qui contient les composants en les quantités suivantes :
| Composants | Quantités (% en poids) |
|---|---|
| Pr, calculé comme Pr₂O₃ | 0,003 - 0,006 |
| Fe, calculé comme Fe₂O₃ | 0,04 - 0,25 |
| Tb, calculé comme Tb₂O₃ | 0,0005 - 0,004 |
| Mn, calculé comme Mn₂O₃ | 0,001 - 0,007 |

13. Composition selon l'une quelconque des revendications 1 à 12, qui contient 4 à 8 % en poids de Y₂O₃.

14. Composition selon l'une quelconque des revendications 1 à 13, qui contient jusqu'à 1 % en poids d'Al₂O₃.

15. Composition selon l'une quelconque des revendications 1 à 14, qui se compose de 98,0 à 99,9999 % en poids de poudre de ZrO₂ nanoscopique.

16. Composition selon la revendication 15, dans laquelle la poudre de ZrO₂ nanoscopique présente une taille des particules primaires comprise entre 5 et 50 nm et une surface spécifique ≥ 100 m²*g⁻¹.

17. Composition selon l'une quelconque des revendications 1 à 16, qui peut être préparée par un procédé pour lequel une poudre à base de ZrO₂ est recouverte d'une source des composants Pr, Fe, Tb et Mn.

18. Composition selon la revendication 17, dans laquelle l'application s'effectue par enduction dans un lit fluidisé.

19. Corps moulé, qui contient la composition selon l'une quelconque des revendications 1 à 18.

20. Corps moulé selon la revendication 19, présent comme ébauche, pièce moulée dentaire ou restauration dentaire.

21. Corps moulé selon la revendication 19 ou 20, qui est multicolore.

22. Corps moulé selon la revendication 21, dans lequel la polychromie est produite par des couches teintées différemment.

23. Corps moulé selon la revendication 22, qui possède au moins 2 et de préférence jusqu'à 8 couches de teintes différentes.

24. Corps moulé selon la revendication 22 ou 23, dans lequel au moins une couche contient la composition selon l'une quelconque des revendications 1 à 18, et en particulier la composition selon l'une quelconque des revendications 9 à 12.

25. Procédé de fabrication d'ébauches et de pièces moulées dentaires, qui contiennent des composés donnant des couleurs et sont définis selon les revendications 20 à 24, dans lequel
a) une poudre d'oxyde contenant du ZrO₂ est recouverte d'une substance donnant des couleurs, une source de composants Pr, Fe, Tb et Mn étant utilisée comme substance donnant des couleurs,
b) la poudre recouverte est le cas échéant triée et le cas échéant versée dans un moule de pressage,
c) la poudre teintée est pressée pour donner un corps moulé, et
d) le corps moulé pressé est fritté pour donner une ébauche et
e) le cas échéant, la pièce moulée dentaire est formée à partir de cette dernière.

26. Procédé selon la revendication 25, **caractérisé en ce qu'**après l'étape a), d'autres poudres d'oxyde non colorées sont recouvertes d'au moins une autre substance donnant des couleurs et sont versées dans un moule de pressage l'une après l'autre.

27. Procédé selon la revendication 25 ou 26, **caractérisé en ce que** des solutions aqueuses de sels métalliques donnant des couleurs des éléments d ou f du système périodique sont utilisées comme substances donnant des couleurs.

28. Procédé selon l'une quelconque des revendications 25 à 27, **caractérisé en ce que** des hydrates de nitrate ou des hydrates de chlorures sont utilisés comme substances donnant des couleurs.

29. Procédé selon l'une quelconque des revendications 25 à 28, **caractérisé en ce que** Pr(NO₃)₃*5H₂O, Fe(NO₃)₃*9H₂O, Tb(NO₃)₃*5H₂O ou CrCl₃*6H₂O sont utilisés comme substances donnant des couleurs.

30. Procédé selon l'une quelconque des revendications 25 à 29, **caractérisé en ce que** les substances donnant des couleurs sont dissoutes dans l'eau avec un liant soluble dans l'eau et sont homogénéisées.

31. Procédé selon l'une quelconque des revendications 25 à 30, **caractérisé en ce que** l'application relative aux poudres d'oxyde s'effectue dans un réacteur à lit fluidisé.

32. Procédé selon l'une quelconque des revendications 25 à 31, **caractérisé en ce que** les poudres d'oxyde sont dotées d'oxydes stabilisateurs de Ce, Y, Al, Ca et/ou de Mg.

33. Procédé selon l'une quelconque des revendications 25 à 32, **caractérisé en ce que** le pressage pour donner les corps moulés s'effectue à des pressions de 50 à 500 MPa.

34. Procédé selon l'une quelconque des revendications 25 à 33, **caractérisé en ce que** le corps moulé est d'abord pré-fritté.

35. Procédé selon l'une quelconque des revendications 25 à 34, **caractérisé en ce que** le corps moulé est d'abord pré-fritté à une température allant de 800 à 1300 °C pour donner une ébauche.

36. Procédé selon l'une quelconque des revendications 34 ou 35, **caractérisé en ce que** la durée du processus de pré-frittage va de 1 à 4 heures.

37. Procédé selon l'une quelconque des revendications 34 à 36, **caractérisé en ce que** la pièce moulée dentaire est fabriquée à partir de l'ébauche pré-frittée au moyen d'un procédé par enlèvement de matière et est frittée à la densité maximale à une température allant de 1200 à 1600 °C.

38. Procédé selon l'une quelconque des revendications 25 à 37, **caractérisé en ce que** la durée du frittage à la densité maximale va de 5 minutes à 2 heures.

39. Procédé selon l'une quelconque des revendications 25 à 38, **caractérisé en ce qu'**une poudre qui se compose de 98,0 à 99,9999 % en poids en poudre de ZrO₂ nanoscopique est utilisée comme céramique d'oxyde pour la fabrication des corps moulés et des ébauches.

40. Procédé selon la revendication 39, **caractérisé en ce que** la poudre de ZrO₂ nanoscopique présente une surface spécifique de ≥ 100 m²g⁻¹ et contient de 0,0001 à 2 % en poids d'un autre composant oxydique.

41. Procédé selon la revendication 40, **caractérisé en ce que** comme autre composant oxydique sont utilisés un composé de ZrO₂ avec une surface spécifique < 100 m²g⁻¹, un composé teinté de ZrO₂ ou un oxyde donnant des couleurs ou un mélange d'oxydes donnant des couleurs.

42. Procédé selon l'une quelconque des revendications 39 à 41, **caractérisé en ce que** la pièce moulée dentaire est fabriquée à partir de l'ébauche pré-frittée au moyen d'un procédé par enlèvement de matière et est frittée à la densité maximale à une température allant de 1000 à 1250 °C.

43. Utilisation de la composition selon l'une quelconque des revendications 1 à 18 comme matériau dentaire.

44. Utilisation de la composition selon l'une quelconque des revendications 1 à 18 ou du corps moulé selon l'une quelconque des revendications 19 à 24 destinée à la fabrication de restaurations dentaires.

45. Utilisation selon la revendication 44, la restauration dentaire étant une couronne et en particulier un bridge.
